(19) Europäisches Patentamt
European Patent Office
Office européen des brevets

(11) Veröffentlichungsnummer: **0 209 763**
**A1**

(12) # EUROPÄISCHE PATENTANMELDUNG

(21) Anmeldenummer: 86108916.7

(22) Anmeldetag: 01.07.86

(51) Int. Cl.[4]: C 07 D 265/30
A 01 N 43/84

(30) Priorität: 05.07.85 DD 278323
05.07.85 DD 278325

(43) Veröffentlichungstag der Anmeldung:
28.01.87 Patentblatt 87/5

(84) Benannte Vertragsstaaten:
AT BE CH DE FR GB IT LI LU NL SE

(71) Anmelder: Institut für Pflanzenschutzforschung
Kleinmachnow der A.d.L. der DDR
Stahnsdorfer Damm 81
DDR-1532 Kleinmachnow(DD)

(72) Erfinder: Banasiak, Lothar, Dr.
Albert-Klink-Strasse 24
DDR-1500 Potsdam(DD)

(72) Erfinder: Leuner, Brita, Dr.
Karl-Marx-Strasse 123
DDR-1532 Kleinmachnow(DD)

(72) Erfinder: Lyr, Horst, Prof. Dr.
Georg-Herwegh-Strasse 8
DDR-1300 Eberswalde(DD)

(72) Erfinder: Nega, Eva
Johannes-Kepler-Platz 4
DDR-1502 Potsdam-Babelsberg(DD)

(72) Erfinder: Sunkel, Marianne
Galileistrasse 83
DDR-1502 Potsdam-Babelsberg(DD)

(74) Vertreter: Waldman, Ralph David
Schering Agrochemicals Limited Industrial Property
Department Chesterford Park Research Station
Saffron Walden Essex CB10 1XL(GB)

(54) **Morpholinio-carbonsäureester- und Morpholinio-alkylphenylethersalze, Verfahren zu ihrer Herstellung und ihre Verwendung als Fungizide.**

(57) Es werden neue N-Alkyl-2,6-dimethylmorpholinio-carbonsäureester- und N-Alkyl-2,6-dimethylmorpholinio-alkyl-phenylether-Salze der allgemeinen Formel I

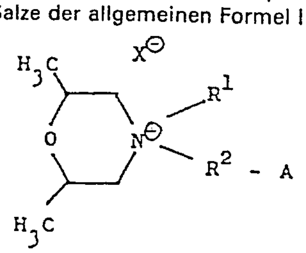

(I)

worin A für die Gruppierung

- CO - Z - $R^3$ oder - O - 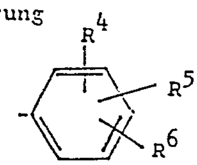 steht,

in der $R^1$ Alkyl mit 6 bis 20 C-Atomen, $R^2$ Alkylen mit 1 bis 6 C-Atomen, $R^3$ Alkyl, substituiertes Alkyl, Alkenyl, Alkinyl, Cycloalkyl, substituiertes Cycloalkyl, Cycloalkylalkyl, Aryl, substituiertes Aryl, Aryl-nieder-alkyl, substituiertes Aryl-nieder-alkyl, Z Sauerstoff oder Schwefel $R^4$, $R^5$ und $R^6$ unabhängig voneinander gleich oder verschieden Wasserstoff, Alkyl, Alkoxy, Acyl, Halogen, Halogen-alkyl, Cycloalkyl, Aryl-nieder-alkyl, Aryl, Nitro, Cyano, Thiocyanato, NHCOR', COOR' und/oder CONR'R'' und $X^-$ das Anion einer Säure bedeuten, Verfahren zu ihrer Herstellung und ihre Verwendung als Fungizide zur Bekämpfung von pilzlichen Schaderregern in der Landwirtschaft und im Gartenbau beschrieben. Zusätzlich besitzen diese Verbindungen auch pflanzenwachstumsregulierende Eigenschaften.

EP 0 209 763 A1

Croydon Printing Company Ltd

Die Erfindung betrifft neue N-Alkyl-2,6-dimethylmorpholinio-carbonsäureester- und N-Alkyl-2,6-dimethylmorpholinio-alkyl-phenylether-Salze, ihre Herstellung und ihre Verwendung als fungizide Mittel in der Landwirtschaft und im Gartenbau mit zusätzlichen pflanzenwachstumsregulierenden Eigenschaften.

Es ist bekannt, N-Alkylmorpholine und ihre Salze sowie ihre Molekül- und Additionsverbindungen als Fungizide zu verwenden (DE-PS 1 164 152, DE-PS 1 173 722, DE-PS 24 61 513).
Bekannt ist ferner, daß quaternäre Ammoniumverbindungen von langkettigen N-Alkyl-2,6-dimethylmorpholinen mit niederen Alkyl-, Alkenyl-, Alkoxyalkyl- oder Aralkyl-Substituenten fungizid wirksam sind (DE-PS 1 167 588; Angewandte Chemie 77 (1965), S. 327-333). Ferner sind Mittel bekannt, die substituierte N-Benzyl- oder Alkoxymethyl-2,6-dimethylmorpholinium-Salze als Wirkstoffe zur Bekämpfung von pilzlichen Schaderregern enthalten (DD 134 037, DD 134 474, DD 140 403). Es ist weiterhin bekannt, daß Salze von Morpholinocarbonsäureestern mit langkettigen Alkansulfon- oder Alkancarbonsäuren fungizide Eigenschaften aufweisen (DD 201 371).

Es ist weiterhin bekannt, daß Morpholinoalkylalkylether, -4-alkylcyclohexylether und -4-alkylarylether fungizide Eigenschaften aufweisen (DE-PS 1 190 724; Angewandte Chemie 77 (1965), S. 327-333; Angewandte Chemie 92 (1980), S. 176-181). Ferner ist bereits bekannt, 2,3-Dihydro-6-methyl-5-phenylarbamoyl-1,4-oxathiin-4,4-dioxid (Oxycarboxin), N,N'-Bis-(1-formamido-2,2,2-trichlorethyl-piperazin (Triforine) oder Zink-ethylen-bis-dithiocarbamat (Zineb) als Wirkstoffe in fungiziden Mitteln zur Bekämpfung von pilzlichen Pflanzenkrankheiten zu verwenden (The Pesticide Manual, British Crop Protection Council; London, 1984).

Die Wirkung der genannten Verbindungen ist jedoch in bestimmten Indikationsbereichen, insbesondere bei niedrigen Aufwandmengen und -konzentrationen, nicht immer voll befriedigend. Außerdem zeigen sie eine recht hohe Selektivität gegenüber bestimmten Arten von pilzlichen Schaderregern, wodurch eine breite Anwendung dieser Mittel stark eingeschränkt wird. Nachteilig ist weiterhin, daß die Pflanzenverträglichkeit dieser Verbindungen in vielen Fällen nicht ausreichend ist.

Der Erfindung liegt die Aufgabe zugrunde, neue N-Alkyl-dimethylmorpholinio-carbonsäureester-Salze und diese enthaltende fungizide Mittel mit guter Wirksamkeit und breitem Wirkungsspektrum sowie einer möglichst hohen Pflanzenverträglichkeit und zusätzlichen pflanzenwachstumsreguliereden Eigenschaften bereitzustellen.

Zur Lösung dieser Aufgabe werden Verbindungen vorgeschlagen, die dadurch gekennzeichnet sind, daß sie mindestens ein N-Alkyl-2,6-dimethylmorpholinio-carbonsäureester- oder N-Alkyl-2,6-dimethylmorpholinio-alkylphenylether-Salz der allgemeinen Formel I,

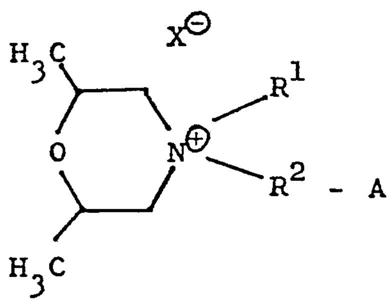

(I) ,

worin A für die Gruppierung

- CO - Z - $R^3$ oder - O - 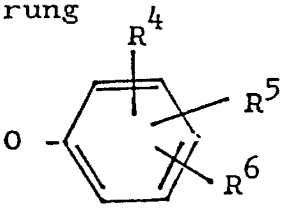 steht,

in der

$R^1$ geradkettiges oder verzweigtes Alkyl mit 6 bis 20 C-Atomen,

$R^2$ geradkettiges oder verzweigtes Alkylen mit 1 bis 6 C-Atomen,

$R^3$ geradkettiges oder verzweigtes Alkyl mit 1 bis 20 C-Atomen,
ein durch Halogen, Hydroxy, Alkoxy mit 1 bis 4 C-Atomen, Dialkylamino mit 2 bis 16 C-Atomen, Nitro und/oder Cyano substituiertes geradkettiges oder verzweigtes Alkyl mit 1 bis 6 C-Atomen,
ein gegebenenfalls durch Halogen substituiertes Alkenyl mit 2 bis 6 C-Atomen,
Alkinyl mit 3 bis 6 C-Atomen,
Cycloalkyl mit 3 bis 12 Kohlenstoffatomen, wobei der Cycloalkylrest gegebenenfalls durch ein oder mehrere Alkylreste mit 1 bis 7 C-Atomen substituierte sein kann,
Cycloalkylalkyl mit 4 bis 10 C-Atomen,
Aryl, welches einfach oder mehrfach, gleich oder verschieden durch geradkettiges oder verzweigtes Alkyl mit 1 bis 6 C-Atomen, Alkoxy mit 1 bis 4 C-Atomen, Aryl-nieder-alkyl mit 7 bis 12 C-Atomen, Acyl mit 1 bis 4 C-Atomen, Halogen, Aryl, Nitro und/oder Cyano substituiert sein kann,
Aryl-nieder-alkyl mit 7 bis 12 C-Atomen, welches einfach oder mehrfach, gleich oder verschieden durch geradkettiges oder verzweigtes Alkyl mit 1 bis 6 C-Atomen, Alkoxy mit 1 bis 4 C-Atomen, Halogen, Halogenalkyl mit 1 bis 4 C-Atomen, Nitro und/oder Cyano substituiert sein kann,

$R^4$, $R^5$ und $R^6$ unabhängig voneinander gleich oder verschieden Wasserstoff, geradkettiges oder verzweigtes Alkyl mit 1 bis 6 C-Atomen, Alkoxy mit 1 bis 4 C-Atomen, Acyl mit 1 bis 4 C-Atomen Halogen, Halogenalkyl mit 1 bis 4 C-Atomen, Cycloalkyl mit 3 bis 7 C-Atomen, Aryl-nieder-alkyl mit 7 bis 12 C-Atomen, Aryl, Nitro, Cyano, Thiocyanato, NHCOR', COOR' und/oder CONR'R'', wobei R' und R'' unabhängig voneinander für Wasserstoff, geradkettiges oder verzweigtes Alkyl mit 1 bis 6 C-Atomen, Aryl oder Aryl-nieder-alkyl mit 7 bis 12 C-Atomen steht,

Z Sauerstoff oder Schwefel und

$X^{\ominus}$ das Anion einer nicht phytotoxischen Säure bedeuten, neben den üblichen Lösungsmitteln, Trägerstoffen und/oder Formulierungshilfsstoffen enthalten.

Die erfindungsgemäßen Verbindungen können in zwei verschiedenen geometrischen Strukturen als N-Alkyl-2,6-cis-dimethylmorpholinio-carbonsäureester- und N-Alkyl-2,6-cis-dimethylmorpholinio-alkyl-phenylether-Salz bzw. N-Alkyl-2,6-trans-dimethylmorpholinio-carbonsäureester- und N-Alkyl-2,6-trans-dimethylmorpholinio-alkyl-phenylether-Salz oder als die jeweiligen Gemische dieser beiden Isomeren vorliegen. Für die fungizide Anwendung kann man sowohl das Isomerengemisch, wie es bei der Synthese anfällt, als auch die einzelnen Isomeren verwenden.

Überraschenderweise wurde gefunden, daß die erfindungsgemäßen N-Alkyl-2,6-dimethylmorpholinio-carbonsäureester- und N-Alkyl-2,6-dimethylmorpholinio-alkyl-phenylether-Salze der allgemeinen Formel I eine starke fungizide Wirksamkeit und ein breites Wirkungsspektrum besitzen und sich insbesondere zur Bekämpfung von phytopathogenen Pilzen an Kulturpflanzen und pflanzlichen Vorratsgütern eignen. Die Wirkstoffe zeigen eine gute Pflanzenverträglichkeit bei den zur Bekämpfung von Pflanzenkrankheiten erforderlichen Aufwandmengen. Zusätzlich können die erfindungsgemäßen Verbindungen mit ihren wachstumsregulierenden Eigenschaften Kulturpflanzen in gewünschter Weise positiv beeinflussen.

Die N-Alkyl-2,6-dimethylmorpholinio-carbonsäureester- und N-Alkyl-2,6-dimethylmorpholinio-alkyl-phenylether-Salze der allgemeinen Formel I werden erhalten, indem man ein N-Alkyl-2,6-dimethylmorpholin der Formel II,

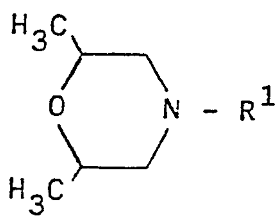

(II)

worin $R^1$ die in der allgemeinen Formel I angegebene Bedeutung besitzt,
mit einer Verbindung der Formel III,

$$X - R^2 - A \qquad (III)$$

worin $R^2$ und A die in der allgemeinen Formel I angegebene Bedeutung besitzen und X für Halogen steht,
umsetzt
oder alternativ einen 2,6-Dimethylmorpholino-carbonsäureester oder 2,6-Dimethylmorpholino-alkyl-phenylether der Formel IV,

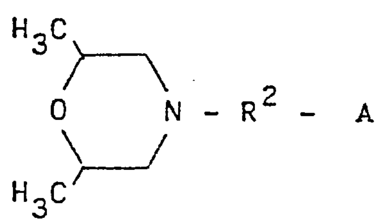

(IV)

worin $R^2$ und A die in der allgemeinen Formel I angegebene Bedeutung besitzen,

mit einer Verbindung der Formel V,

$$R^1 - X \qquad (V)$$

worin $R^1$ die in der allgemeinen Formel I angegebene Bedeutung besitzt und X für Halogen steht,
umsetzt.

N-Alkyl-2,6-dimethylmorpholine der Formel II sind z.B. n-Decyl-, n-Dodecyl-, n-Tridecyl-, iso-Tridecyl-, 1,5,9-Trimethyldecyl-, n-Pentadecyl- oder n-Didecyl-2,6-dimethylmorpholin.

Halogencarbonsäureester der Formel III sind beispielsweise Chloressigsäure-$C_1$-$C_{20}$-alkylester, Chloressigsäure-4-chlorbutylester, Chloressigsäure-4-hydroxybutylester, Choressigsäure-2-ethoxyethylester, Choressigsäure-2-cyanoethylester, Chloressigsäure-2-nitroethylester, Chloressigsäure-cyclohexylester, 2-Brompropionsäuremethylester, 2-Brompropionsäureallylester, 2-Brompropionsäure-1-naphthylester, Chloressigsäure-phenylester, Thiol-chloressigsäurephenylester, 2-Brombuttersäuremethylester, 2-Bromcapronsäure-benzylester, Chloressigsäure-4-chlorphenylester, Chloressigsäure-4-tert.-butylphenylester, Chloressigsäure-3,4-dichlorphenylester, Chloressigsäure-2,6-dimethylphenylester, Chloressigsäure-2,6-dibrom-4-nitrophenylester, Chloressigsäure-2,6-dibrom-4-cyanophenylester, Chloressigsäure-2,6-dichlorbenzylester, Chloressigsäure-4-cyanobenzylester, Chloressigsäure-4-nitrophenylester oder das Chloracetat-Anion.

Halogenalkyl-phenylether der Formel III sind beispielsweise 2-Bromethyl-phenylether, 2-Chlorethyl-4-tert.-butylphenylether, 2-Bromethyl-4-nitrophenylether, 2-Bromethyl-4-cyanophenylether, 2-Bromethyl-2,6-dichlorphenylether, 2-Bromethyl-3,4-dichlorphenylether, 2-Bromethyl-2,6-dichlor-4-cyanophenylether, 2-Bromethyl-3,5-dichlorphenylether, 2-Bromethyl-4-benzylphenylether, 2-Bromethyl-4-acetamidophenylether, 3-Brompropyl-phenylether, 3-Brompropyl-4-tert.-butylphenylether, 3-Brompropyl-2,6-dimethylphenylether oder 3-Brompropyl-2,6-dibrom-4-thiocyanatophenylether.

2,6-Dimethylmorpholino-carbonsäureester der Formel IV sind z.B. 2,6-Dimethylmorpholino-essigsäure-$C_1$-$C_{20}$-alkylester, -essigsäure-phenylester, -thiol-essigsäure-phenylester, -essigsäure-4-chlorphenylester, -essigsäure-4-nitrophenylester, -essigsäure-3,4-dichlorphenylester, -essigsäure-2,6-dibrom-4-cyanophenylester, -essigsäurebenzylester, essigsäure-2,6-dichlorbenzylester, -2-propionsäure-methylester, -2-buttersäuremethylester oder -acetat, wenn $R^3$ nicht vorhanden ist.

2,6-Dimethylmorpholino-alkyl-phenylether der Formel IV sind z.B. 2-(2,6-dimethylmorpholino)-ethyl-phenylether, -4-cyanophenylether, -4-tert.-butylphenylether, -4-nitrophenylether, -3,4-dichlorphenylether, -2,6-dichlor-4-cyanophenylether, 3-(2,6-dimethylmorpholino)-propyl-phenylether, -4-tert.-butylphenylether, -3,5-dichlorphenylether, -2,6-dimethylphenylether oder -2,6-dibrom-4-thiocyanato-phenylether.

Alkylhalogenide der Formel V sind beispielsweise n-Decylchlorid, n-Dodecylchlorid, n-Tridecylchlorid, iso-Tridecylchlorid, 1,5,9-Trimethyldecylchlorid, Pentadecylbromid oder Didecylbromid.

Die Ausgangsverbindungen zur Herstellung der erfindungsgemäßen Verbindungen sind an sich bekannt oder können nach an sich bekannten Verfahren hergestellt werden.

Die Umsetzung zu den erfindungsgemäßen Verbindungen der Formel I werden gegebenenfalls in Gegenwart eines Lösungs- oder Verdünnungsmittels bei einer Temperatur im Bereich zwischen 10 und 180 °C, vorzugsweise zwischen 30 und 150 °C, durchgeführt. Die Ausgangsstoffe der Formel II bzw. der Formel IV werden in stöchiometrischen Mengen mit einer Verbindung der Formel III bzw. der Formel V oder bevorzugt mit einem Oberschuß von 10 bis 100 % an einer Verbindung der Formel III bzw. der Formel V über die stöchiometrische Menge hinaus, bezogen auf die Ausgangsstoffe der Formel II bzw. der Formel IV, umgesetzt.

Als bevorzugte Lösungs- oder Verdünnungsmittel können beispielsweise aliphatische oder aromatische, gegebenenfalls halogenierte Kohlenwasserstoffe, wie n-Pentan, Cyclohexan, Benzen, Toluen, Chlorbenzen, Chloroform oder Methylenchlorid; aliphatische Ketone, wie Aceton, Methylethylketon oder Cyclohexanon; Ether, wie Diethylether, Tetrahydrofuran oder Dioxan; Alkohole, wie Methanol, Ethanol, Propanole, Butanole oder Hexanole; Nitrile, wie Acetonitril; Ester, wie Essigsäuremethylester; Amide, wie Dimethylformamid, Dimethylacetamid oder N-Methyl-pyrrolidon; Dimethylsulfoxid oder Wasser oder Gemische dieser Lösungsmittel verwendet werden.

Die Isolierung der erfindungsgemäßen Verbindungen der allgemeinen Formel I aus den Reaktionsmischungen ist nicht unbedingt erforderlich, da sie auch ohne weitere Reinigungsoperation zur Herstellung fungizider Zubereitungen einsetzbar sind.

Die erfindungsgemäßen Wirkstoffe der allgemeinen Formel I besitzen eine starke Wirksamkeit gegen Mikroorganismen und können dementsprechend zur Bekämpfung von pilzlichen Schaderregern in der Landwirtschaft und im Gartenbau Verwendung finden. Mit den Wirkstoffen können an Pflanzen oder Pflanzenteilen auftretende unerwünschte Pilze bekämpft werden. Die Wirkstoffe der allgemeinen Formel I eignen sich ferner als Beizmittel zur Behandlung von Saatgut und Pflanzenstecklingen zum Schutz vor Pilzinfektionen und können gegen im Erdboden auftretenden phytopathogene Pilze eingesetzt werden. Zusätzlich beeinflussen die Wirkstoffe bei ihrer Anwendung die Wachstumsprozesse von Kulturpflanzen in positiver Weise.

Die Ausgangsverbindungen zur Herstellung der erfindungsgemäßen Verbindungen sind an sich bekannt oder können nach an sich bekannten Verfahren hergestellt werden.

Die erfindungsgemäßen Wirkstoffe eignen sich besonders zur Verhütung und Heilung von Pflanzenkrankheiten, die durch Pilze verursacht werden, wie z.B. Erysiphe graminis (Getreidemehltau), Erysiphe cichoracearum (Gurkenmehltau), Erysiphe polygoni (Bohnenmehltau), Podosphaera leucotricha (Apfelmehltau), Sphaerotheca pannosa (Rosenmehltau), Uncinula necator (Rebenmehltau); Rostkrankheiten, wie solche der Gattungen Puccinia, Uromyces oder Hemileia, insbesondere Puccinia graminis (Getreideschwarzrost), Puccinia coronata (Haferkronenrost), Puccinis sorghi (Maisrost), Puccinia recondita (Getreidebraunrost), Uromyces fabae (Buschbohnenrost), Hemileia vastatrix (Kaffeerost); Botrytis cinerea an Reben und Erdbeeren; Monilia fructigena an Äpfeln; Plasmopara viticola an Reben; Mycosphaerella musicola an Bananen; Corticum salmonicolor an Hevea; Ganoderma pseudoferreum an Hevea; Exobasidium vexans an Tee; Phytophthora infestans an Kartoffeln und Tomaten; Alternaria solani an Tomaten. Ferner sind verschiedene dieser Wirkstoffe auch unterschiedlich wirksam gegen phytopathogene Pilze, wie z.B. Ustilago avenae (Flugbrand), Ophiobolus graminis (Getreidefußkrankheiten), Septoria nodorum (Getreideblatt- und Spelzenbräune), Venturia inaequalis (Apfelschorf) sowie weitere pilzliche Krankheitserreger, wie Rhizoctonia, Tilletia, Helminthosporium, Peronospora, Pythium, Alternaria, Mucor, Sclerotinia, Fusarium, Pseudocercosporella und Cladosporium.

Besonders interessant sind die erfindungsgemäßen Wirkstoffe für die Bekämpfung einer Vielzahl von Pilzkrankheiten an verschiedenen Kulturpflanzen oder ihren Samen, insbesondere Weizen, Roggen, Gerste, Hafer, Reis, Mais, Baumwolle, Soja, Kaffee, Bananen, Zuckerrohr, Obst, Zierpflanzen im Gartenbau, Gemüse, wie Gurken, Bohnen oder Kürbisgewächse.

Zusätzlich können die erfindungsgemäßen Wirkstoffe mit ihren pflanzenwachtumsregulierenden Eigenschaften die Entwicklung von Kulturpflanzen in gewünschter Weise positiv beeinflussen. Die Wirkungen der Verbindungen sind im wesentlichen von dem Zeitpunkt der Anwendung, bezogen auf das Entwicklungsstadium des Samens oder der Pflanzen, von den auf die Pflanzen oder ihre Umgebung ausgebrachten Wirkstoffmengen und von der Art der Applikation abhängig.

Weiterhin ei ringen die Wirkstoffe auch eine gute Wirksamkeit gegen holzverfärbende und holzzerstörende Pilze, wie z.B. Pullaria pullulans, Apsergillus niger, Polystictus versicolor oder Chaetomium globosum.

Ferner zeigen die erfindungsgemäßen Wirkstoffe der allgemeinen Formel I eine gute Aktivität gegen Schimmelpilze, wie z.B. Penicillium-, Fusarium- oder Aspergillus-Arten, die einen Verderb von hochfeuchtigkeitshaltigen landwirtschaftlichen Produkten oder Verarbeitungsprodukten landwirtschaftlicher Erzeugnisse während der Lagerung oder Zwischenlagerung verursachen. Derartig zu behandelnde Produkte umfassen z.B. Äpfel, Apfelsinen, Mandarinen, Zitronen, Pampelmusen, Erdnüsse, Getreide und Getreideprodukte oder Hülsenfrüchte und -schrot.

Die erfindungsgemäßen Wirkstoffe sind neben ihrem breiten fungiziden Wirkungsspektrum auch unterschiedlich gegen phytopathogene Bakterien, wie beispielsweise Xanthomonas- oder Erwinia-Arten, wirksam.

Einige der Wirkstoffe zeigen auch eine Wirkung gegen humanpathogene Pilze, wie z.B. Trichophyten- und Candida-Arten.

Ein Teil der Wirkstoffe der allgemeinen Formel I zeichnet sich neben der protektiven Wirkung durch eine systemische Wirkungsentfaltung aus. So werden sie sowohl über die Wurzel als auch über die Blätter aufgenommen und im Pflanzengewebe transportiert oder über das Saatgut den oberirdischen Teilen der Pflanze zugeführt.

Die erfindungsgemäßen Wirkstoffe sind ferner geeignet zur Bekämpfung resistenter Stämme pilzlicher Schaderreger, die gegen bekannte fungizide Wirkstoffe, wie z.B. Wirkstoffe aus der Gruppe der Dicarboximid-Fungizide, wie beispielsweise 5-Methyl-5-vinyl-3-(3,5-dichlorphenyl)-2,4-dioxo-1,3-oxazolidin (Vinclozolin) oder 5-Methyl-5-methoxymethyl-3-(3,5-dichlorphenyl)-1,3-oxazolidin-2,4-dion (Myclozolin); Wirkstoffe aus der Gruppe der Benzimidazol- oder Thiophanat-Fungizide, wie beispielsweise 1-(n-Butylcarbamoyl)-benzimidazol-2-yl-carbaminsäuremethylester (Benomyl), Benzimidazol-2-yl-carbaminsäuremethylester (Carbendazim) oder 1,2-Bis-(3-ethoxycarbonyl-2-thioureido)-benzen (Thiophanat); Wirkstoffe aus der

Gruppe der Azol-Fungizide, wie beispielsweise 1-(4-Chlorphenoxy-3,3-dimethyl-1-(1H-1,2,4-triazol-1-yl)-butan-2-on (Triadimefon) oder 1-/2'-(2'',4''-Dichlorphenyl)-2'-(propenyloxy)-ethyl/-1,3-imidazol (Imazalil); Wirkstoffe aus der Gruppe der aromatische Kohlenwasserstoffe enthaltenden Fungizide, wie beispielsweise 2,5-Dichlor-1,4-dimethoxy-benzen (Chloroneb) oder 2,6-Dichlor-4-nitro-anilin (Dicloran); Wirkstoffe aus der Gruppe der Acylalanin-Fungizide, wie beispielsweise DL-N-(2,6-dimethylphenyl)-N-(2'-methoxyacetyl)-alanin-methylester (Metalaxyl) oder DL-N-(2,6-dimethylphenyl)-N-(2-furoyl)-alanin-methylester (Furalaxyl) oder Wirkstoffe aus der Gruppe der Pyrimidin-Fungizide, wie beispielsweise 5-Butyl-2-dimethylamino-4-hydroxy-6-methyl-pyrimidin (Dimethirimol) oder 2-Chlorphenyl-4-chlorphenyl-pyrimidin-5-yl-methanol (Fenarimol), Resistenzerscheinungen zeigen.

Die in der allgemeinen Formel I der erfindungsgemäßen Wirkstoffe aufgeführten Anionen $X^-$ sind für die fungizide Wirkung nicht ausschlaggebend.

Die erfindungsgemäßen Wirkstoffe können in die üblichen Formulierungen überführt werden, wie Lösungen, Emulsionen, Spritzpulver, Suspensionen, Pulver, Stäubemittel, Schäume, Pasten, lösliche Pulver, Granulate, mit Wirkstoff imprägnierte Natur- und synthetische Stoffe, Feinstverkapselungen in polymeren Stoffen und in Hüllmassen für Saatgut sowie ULV-Kalt- und Warmneben-Formulierungen.
Die Herstellung dieser Formulierungen kann in bekannter Weise erfolgen, z.B. durch Vermischen oder Vermahlen der erfindungsgemäßen Wirkstoffe der allgemeinen Formel I mit Lösungsmitteln und/oder Trägerstoffen, gegebenenfalls unter Verwendung von oberflächenaktiven Mitteln, wie Emulgiermitteln und/oder Dispergiermitteln.
Als flüssige Lösungsmittel können Aromaten, wie Toluen, Xylen oder Alkylnaphthalene; chlorierte Aromaten oder chlorierte aliphatische Kohlenwasserstoffe, wie Chlorbenzene, Chlorethylene oder Methylenchlorid; aliphatische Kohlenwasserstoffe, wie Cyclohexan oder Paraffine, z.B. Erdölfraktionen; Alkohole, wie Butanole oder Glykole sowie deren Ester und Ether; Ketone, wie Aceton, Methylethylketon,

Methylisobutylketon oder Cyclohexanon; stark polare Lösungsmittel, wie Wasser, Dimethylformamid oder Dimethylsulfoxid verwendet werden. Ebenso können verflüssigte Lösungsmittel, die unter Normalbedingungen gasförmig sind, wie z.B. Aerosol-Treibgase, wie Halogenkohlenwasserstoffe, Propan, Butan, Stickstoff und Kohlendioxid eingesetzt werden.
Als feste Trägerstoffe kommen z.B. natürliche Gesteinsmehle, wie Kaoline, Tonerden, Talkum, Kreide, Quarz, Montmorillonit oder Diatomeenerde und synthetische Gesteinsmehle, wie hochdisperse Kieselsäure, Aluminiumoxid und Silikate in Frage. Als feste Trägerstoffe für Granulate können z.B. gebrochene und fraktionierte natürliche Gesteinsmehle, wie Calcit, Marmor, Bims, Dolomit sowie synthetische Granulate aus anorganischen oder organischen Mehlen sowie Granulate aus organischen Materialien, wie Sägemehl, Cellulosepulver, Baumrinden- und Nußschalenmehl, verwendet werden.
Als Emulgiermittel können z.B. nichtionogene und anionische Emulgatoren, wie Alkali-, Erdalkali- oder Ammoniumsalze von Ligninsulfonsäure, Naphthalensulfonsäuren, Phenolsulfonsäuren, Alkylarylsulfonate, Alkylsulfate, Alkylsulfonate, Alkali- und Erdalkalisalze der Dibutylnaphthalensulfonsäure, Laurylethersulfat, Fettalkoholsulfate, fettsaure Alkali- und Erdalkalisalze, Salze sulfatierter Hexadecanole, Heptdecanole oder Octadecanole, Salze von sulfatierten Fettalkoholglykolethern, Kondensationsprodukte von sulfonierten Naphthalen und Naphthalenderivaten mit Formaldehyd, Kondensationsprodukte des Naphthalens bzw. der Naphthalensulfonsäuren mit Phenol und Formaldehyd, Polyoxyethylen-octylphenolether, Alkylphenolpolyglykolether, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal und Sorbitester, eingesetzt werden.
Als Dispergiermittel kommen beispielsweise Lignin-Sulfitablaugen und Methylcellulose in Betracht.
Es können in den Formulierungen Haftmittel, wie Carboxymethylcellulose, natürliche und synthetische latexförmige Polymere, wie Gummiarabicum, Polyvinylalkohol und Polyvinylacetat, verwendet werden.
Als weitere Zusätze können Farbstoffe und Spurennährstoffe in den

Formulierungen der Wirkstoffe enthalten sein.
Die Formulierungen enthalten im allgemeinen zwischen 0,1 und 95 Masseprozent Wirkstoff, vorzugsweise zwischen 0,5 und 90 Masseprozent.

Das erfindungsgemäße Verfahren zur Bekämpfung von Pilzen ist dadurch gekennzeichnet, daß man fungizide Mittel mit den erfindungsgemäßen Wirkstoffen der allgemeinen Formel I auf Pilze oder die vor Pilzbefall zu schützenden Gegenstände in wirksamer Menge einwirken läßt.

Die erfindungsgemäßen Wirkstoffe können in den Formulierungen oder in den verschiedenen Anwendungsformen mit anderen bekannten Wirkstoffen, wie Fungiziden, Bakteriziden, Insektiziden, Akariziden, Nematiziden, Herbiziden, Wachstumsregulatoren, Pflanzennährstoffen und Bodenstrukturverbesserungsmitteln vermischt und ausgebracht werden. In vielen Fällen erhält man bei der Mischung mit Fungiziden eine Verbreiterung des fungiziden Wirkungsspektrums. Bei einer Anzahl von Mischungen der erfindungsgemäßen Wirkstoffe mit bekannten Fungiziden treten auch synergistische Effekte auf, wobei die fungizide Wirksamkeit des Kombinationsproduktes größer ist als die der addierten Wirksamkeit der Einzelkomponenten.
Fungizide, die mit den erfindungsgemäßen Wirkstoffen kombiniert werden können, ohne dabei die Kombinationsmöglichkeiten einzuschränken, sind beispielsweise:

Schwefel,
Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat und Zinkethylenbisdithiocarbamat,
Tetramethylthiuramsulfide,
Ammoniak-Komplex von Zink-(N,N-ethylen-bis-dithiocarbamat) und N,N'-Polyethylen-bis-(thiocarbamoyl)-disulfid,
Ammoniak-Komplex von Zink-(N,N'-propylen-bis-dithiocarbamat) und N,N'-Propylen-bis-(thiocarbamoyl)-disulfid,

N-Trichlormethylthio-tetrahydrophthalimid,
N-Trichlormethylthio-phthalimid,

N-(1,1,2,2-Tetrachlorethylthio)-tetrahydrophthalimid,

4,6-Dinitro-2-(1-(methylheptyl)-phenylcrotonat,
4,6-Dinitro-2-sek.-butylphenyl-3,3-dimethylacrylat,
4,6-Dinitro-2-sek.-butylphenyl-isopropylcarbonat,

1-(n-Butylcarbamoyl)-benzimidazol-2-yl-carbaminsäuremethylester,
Benzimidazol-2-yl-carbaminsäuremethylester,
2-(Fur-2-yl-)-benzimidazol,
2-(Thiazol-4-yl-)-benzimidazol,
1,2-Bis-(3-methoxycarbonyl-2-thioureido)-benzen,
1,2-Bis-(3-ethoxycarbonyl-2-thioureido)-benzen,
2,3-Dihydro-6-methyl-5-phenylcarbamoyl-1,4-oxathiin,
2,3-Dihydro-6-methyl-5-phenylcarbamoyl-1,4-oxathiin-4,4-dioxid,

Tetrachlor-isophthalsäuredinitril,
2,3-Dichlor-1,4-naphthochinon,
2,3-Dicyano-1,4-dithioanthrachinon,

N-Tridecyl-2,6-dimethylmorpholin bzw. dessen Salze,
N-$C_{10}$-$C_{14}$-Alkyl-2,5- und/oder 2,6-dimethylmorpholin,
N-Cyclododecyl-2,6-dimethylmorpholin bzw. dessen Salze,
N-/3-p-tert.-Butylphenyl)-2-methyl-propyl/-2,6-cis-dimethylmorpholin bzw. dessen Salze,
N,N'-Bis-(1-Formamido-2,2,2-trichlorethyl)-piperazin,
N-(1-Formamido-2,2,2-trichlorethyl)-3,4-dichloranilin,
N-(1-Formamido-2,2,2-trichlorethyl)-morpholin,

5-Butyl-2-ethylamino-4-hydroxy-6-methyl-pyrimidin,
5-Butyl-2-dimethylamino-4-hydroxy-6-methyl-pyrimidin

2,4-Dichlorphenyl-phenyl-pyrimidin-5-yl-methanol,
2-Chlorphenyl-4-chlorphenyl-pyrimidin-5-yl-methanol,
Bis-(4-chlorphenyl)-pyridin-3-yl-methanol,

5-Methyl-5-vinyl-3-(3,5-dichlorphenyl)-1,3-oxazolidin-2,4-dion,
5-Methyl-5-methoxymethyl-3-(3,5-dichlorphenyl)-1,3-oxazolidin-2,4-dion,
3-(3,5-Dichlorphenyl)-N-isopropyl-2,4-dioxo-imidazolidin-1-carboxamid,
N-(3,5-Dichlorphenyl)-1,2-dimethylcyclopropan-1,2-dicarboximid,

1-(4-Chlorphenoxy)-3,3-dimethyl-1-(1,2,4-triazol-1-yl)-butan-2-on
1-(4-Chlorphenoxy)-3,3-dimethyl-1-(1,2,4-triazol-1-yl)-butan-2-ol
1-/2-(2,4-Dichlorphenyl)-2-(propenyloxy)-ethyl/-imidazol
1-/N-Propyl-N-(2,4,6-trichlorphenoxy)-ethyl-carbamoyl/-imidazol
1-/2-(2,4-Dichlorphenyl)-4-ethyl-1,3-dioxolan-2-yl-methyl/-1H-1,2,4-triazol
1-/2-(2,4-Dichlorphenyl)4-n-propyl-1,3-dioxolan-2-yl-methyl/-1H-1,2,4-triazol
1-(2,4-Dichlorphenyl)-2-(1,2,4-triazol-1-yl)-4,4-dimethyl-pentan-3-on

2,5-Dichlor-1,4-dimethoxy-benzen
2,6-Dichlor-4-nitroanilin
Diphenyl

2-Methyl-benzoesäure-anilid
2-Jod-benzoesäure-anilid
2,5-Dimethyl-furan-3-carbonsäureanilid
2,4,5-Trimethyl-furan-3-carbonsäureanilid
N-Cyclohexyl-N-methoxy-2,5-dimethyl-furan-3-carbonsäureamid
3-/3-(3,5-Dimethyl-2-oxycyclohexyl)-2-hydroxyethyl/-glutarimid
N-Dichlorfluormethylthio-N',N'-dimethyl-N-phenyl-schwefelsäure-diamid
DL-N-(2,6-Dimethylphenyl)-N-(2'-methoxyacetyl)-alanin-methylester
DL-N-(2,6-Dimethylphenyl)-N-(2-furoyl)-alanin-methylester
N-(2,6-Dimethylphenyl)-N-chloracetyl-DL-2-aminobutyrolacton

2,4-Dichlor-6-(2-chloranilino)-s-triazin
O,O-Diethyl-phthalimido-phosphonothioat
5-Amino-1-(bis-(dimethylamino)-phosphinyl)-3-phenyl-1,2,4-triazol
O,O-Diethyl-S-benzyl-thiolphosphorsäureester
2-Thio-1,3-dithio-(4,5-b)-chinoxalin)
4-(2-Chlorphenylhydrazono)-3-methyl-5-isoxazolon
Pyridin-2-thiol-1-oxid
8-Hydroxychinolin bzw. dessen Salze
4-Dimethylaminophenyl-diazo-sulfonsäure-Natriumsalz
5-Nitro-isophthalsäure-di-isopropylester

2-Cyan-N-(ethylaminocarbonyl)-2-(methoxyimino)-acetamid
2-Heptadecyl-2-imidazolin-acetat
Dodecyl-guanidinacetat

Die Wirkstoffe können als solche, in Form ihrer Formulierungen oder den daraus durch weiteres Verdünnen bereiteten Anwendungsformen in üblicher Weise, z.B. durch Gießen, Tauchen, Spritzen, Sprühen, Vernebeln, Injizieren, Verschlämmen, Verstreichen, Stäuben, Streuen, Trockenbeizen, Feuchtbeizen, Naßbeizen, Schlämmbeizen oder Inkrustieren, angewendet werden.
Bei der Behandlung von Pflanzenteilen können die Wirkstoffkonzentrationen in den Anwendungsformen in einem größeren Bereich variiert werden. Sie liegen im allgemeinen zwischen 0,0001 und 1 Masseprozent, vorzugsweise zwischen 0,5 und 0,001 Masseprozent. Die Wirkstoffaufwandmengen sind vom spezifischen Anwendungszweck abhängig und liegen im allgemeinen zwischen 0,1 und 3 kg Wirkstoff pro Hektar.
Bei der Saatgutbehandlung werden im allgemeinen Wirkstoffmengen von 0,001 bis 50 g oder mehr je Kilogramm Saatgut, vorzugsweise 0,01 bis 10 g, benötigt.
Zur Konservierung oder Nacherntebehandlung von landwirtschaftlichen Produkten oder Verarbeitungsprodukten landwirtschaftlicher Erzeugnisse betragen die benötigten Wirkstoffmengen 0,01 bis 100 g je Kilogramm Behandlungsgut, vorzugsweise 0,1 bis 50 g.
Bei der Behandlung des Bodens sind Wirkstoffkonzentrationen von 0,0001 bis 0,1 Masseprozent, vorzugsweise von 0,001 bis 0,05 Masseprozent, am Wirkungsort erforderlich.
Die nachfolgenden Beispiele sollen die Erfindung weiter erläutern, ohne sie jedoch einzuschränken und die Wirkung der erfindungsgemäßen Verbindungen der allgemeinen Formel I belegen.

Ausführungsbeispiele

Die nachfolgenden Beispiele erläutern die Herstellung der erfindungsgemäßen Verbindungen.

Beispiel 1

N-iso-Tridecyl-2,6-dimethylmorpholinio-essigsäuremethylester-chlorid

30 g N-iso-Tridecyl-2,6-dimethylmorpholin und 10,9 g Chloressigsäuremethylester werden unter Zugabe einer katalytischen Menge Natriumjodid in 100 ml Acetonitril 20 Stunden zum Rückfluß erwärmt. Man kühlt ab und destilliert das Lösungsmittel im Vakuum ab. Das Produkt wird mit n-Hexan dirigiert. Nach Einengen im Vakuum erhält man 38 g eines gelbbraunen viskosen Öles (Verbindung Nr. 5) IR-Spektrum (Film: C = O - Absorption 1740 $cm^{-1}$.

Beispiel 2

N-iso-Tridecyl-2,6-dimethylmorpholinio-essigsäure-3,4-dichlorphenylester-chlorid

30 g N-iso-Tridecyl-2,6-dimethylmorpholin und 24 g Chloressigsäure-3,4-dichlorphenylester werden in 100 ml n-Butanol unter Zugabe einer katalytischen Menge Natriumjodid 16 Stunden zum Rückfluß erwärmt. Man kühlt ab und destilliert das Lösungsmittel im Vakuum ab. Das zurückbleibende Produkt wird in wenig Diethylether gelöst und mit n-Hexan bis zur vollständigen Abscheidung als ölige Phase versetzt. Nach Einengen im Vakuum erhält man 48 g eines dunkelbraunen Harzes (Verbindung Nr. 55)

IR-Spektrum (Film): C = O - Absorption 1740 $cm^{-1}$

C = C - Absorption (Aromat) 1590 $cm^{-1}$

Beispiel 3

2-(N-iso-Tridecyl-2,6-dimethylmorpholinio)-propionsäure-methylester-chlorid

20 g 2-(2,6-Dimethylmorpholino)-propionsäuremethylester und 22 g iso-Tridecylchlorid (Gemisch verschiedener $C_{11}$ - $C_{14}$-Alkyl-

chloride, das 60 bis 70 % n-Tridecylchlorid enthält) in 100 ml Dimethylformamid werden 10 Stunden zum Rückfluß erhitzt. Nach dem Abkühlen wird das Lösungsmittel im Vakuum abdestilliert. Das Produkt wird in wenig Aceton gelöst und mit n-Hexan bis zur vollständigen Abscheidung als ölige Phase versetzt. Beim Einengen im Vakuum erhält man 19 g eines hellbraunen Harzes (Verbindung Nr. 8)
IR-Spektrum (Film): C = O - Absorption 1735 $cm^{-1}$

Beispiel 4

3-(N-iso-Tridecyl-2,6-dimethylmorpholinio)-propyl-2,6-dimethylphenylether-bromid

15 g N-iso-Tridecyl-2,6-dimethylmorpholin und 12,2 g 3-Brompropyl-2,6-dimethylphenylether in 50 ml Acetonitril werden unter Zusatz einer katalytischen Menge Natriumjodid 23 Stunden zum Rückfluß erhitzt. Nach dem Abkühlen wird das Lösungsmittel im Vakuum abdestilliert. Man nimmt in wenig Aceton auf und versetzt die Lösung bis zur vollständigen Abscheidung mit n-Hexan. Die ölige Phase wird abgetrennt und im Vakuum von Lösungsmittelresten befreit. Man erhält 21 g eines gelbbraunen viskosen Öles (Verbindung Nr. 107).

Beispiel 5

2-(N-iso-Tridecyl-2,6-dimethylmorpholinio)-ethyl-3,4-dichlorphenylether-chlorid

17 g 2(2,6-Dimethylmorpholino)-ethyl-3,4-dichlorphenylether und 11 g iso-Tridecylchlorid (Gemisch verschiedener $C_{11}$-$C_{14}$-Alkylhalogenide, das 60 bis 70 % n-Tridecylchlorid enthält) in 50 ml Dimethylformamid werden 10 Stunden zum Rückfluß erwärmt. Man läßt abkühlen und destilliert das Lösungsmittel im Vakuum ab. Der verbleibende Rückstand wird in wenig Aceton gelöst und mit n-Hexan bis zur vollständigen Abscheidung als ölige Phase versetzt.

Das Produkt wird abgetrennt und im Vakuum von Lösungsmittelresten befreit. Man erhält 19 g eines braunen harzartigen Produktes (Verbindung Nr. 106).

In entsprechender Weise werden die nachfolgend aufgeführten Verbindungen der allgemeinen Formel I hergestellt, die in der Regel gelbe bis braune viskose Öle oder Harze darstellen, in polaren Lösungsmitteln, wie z.B. Alkohole, Aceton, Dimethylformamid und Dimethylsulfoxid, gut löslich sind und durch IR-Spektren charakterisiert werden.

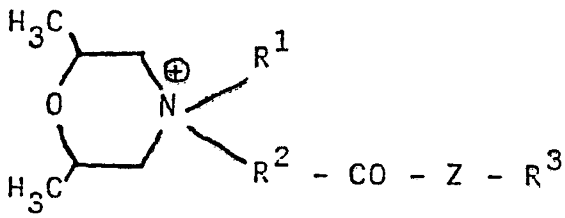

$X^{\ominus}$ (I)

| Nr. | $R^1$ | $R^2$ | Z | $R^3$ | X |
|---|---|---|---|---|---|
| 1 | n-decyl | $CH_2$ | O | methyl | Cl |
| 2 | n-dodecyl | $CH_2$ | O | methyl | Cl |
| 3 | n-tridecyl | $CH_2$ | O | methyl | Cl |
| 4 | 1,5,9-trimethyl-decyl | $CH_2$ | O | methyl | Cl |
| 5 | iso-tridecyl | $CH_2$ | O | methyl | Cl |
| 6 | n-pentadecyl | $CH_2$ | O | methyl | Br |
| 7 | n-didecyl | $CH_2$ | O | methyl | Br |
| 8 | iso-tridecyl | $CH(CH_3)$ | O | methyl | Cl |
| 9 | iso-tridecyl | $CH(C_2H_5)$ | O | methyl | Br |
| 10 | iso-tridecyl | $C(CH_3)_2$ | O | methyl | Br |
| 11 | iso-tridecyl | $C(C_2H_5)_2$ | O | methyl | Br |
| 12 | iso-tridecyl | $CH(C_5H_{11})$ | O | methyl | Br |
| 13 | iso-tridecyl | $(CH_2)_3$ | O | methyl | Cl |
| 14 | iso-tridecyl | $CH_2$ | O | n-butyl | Cl |
| 15 | iso-tridecyl | $CH_2$ | S | n-butyl | Cl |
| 16 | iso-tridecyl | $CH_2$ | O | 2-ethylhexyl | Cl |
| 17 | iso-tridecyl | $CH_2$ | O | n-dodecyl | Cl |
| 18 | iso-tridecyl | $CH_2$ | O | iso-octadecyl | Cl |
| 19 | iso-tridecyl | $CH_2$ | O | 4-hydroxybutyl | Cl |
| 20 | iso-tridecyl | $CH_2$ | O | 2-ethoxyethyl | Cl |
| 21 | iso-tridecyl | $CH_2$ | O | 4-chlorbutyl | Cl |
| 22 | iso-tridecyl | $CH_2$ | O | 2,2,2-trichlor-ethyl | Cl |
| 23 | iso-tridecyl | $CH_2$ | O | 2-nitroethyl | Cl |

| Nr. | $R^1$ | $R^2$ | Z | $R^3$ | X |
|---|---|---|---|---|---|
| 24 | iso-tridecyl | $CH_2$ | O | 2-cyanethyl | Cl |
| 25 | iso-tridecyl | $CH_2$ | O | cyclopentyl | Cl |
| 26 | iso-tridecyl | $CH_2$ | S | cyclohexyl | Cl |
| 27 | iso-tridecyl | $CH_2$ | O | 2,6-dimethylcyclohexyl | Cl |
| 28 | iso-tridecyl | $CH_2$ | O | cyclooctyl | Cl |
| 29 | iso-tridecyl | $CH_2$ | O | cyclododecyl | Cl |
| 30 | iso-tridecyl | $CH_2$ | O | 5-(2-ethylcyclohexyl)-pentyl | Cl |
| 31 | iso-tridecyl | $CH_2$ | O | cyclododecylmethyl | Cl |
| 32 | iso-tridecyl | $CH_2$ | O | allyl | Cl |
| 33 | iso-tridecyl | $CH_2$ | O | crotyl | Cl |
| 34 | iso-tridecyl | $CH_2$ | O | propargyl | Cl |
| 35 | iso-tridecyl | $CH_2$ | O | phenyl | Cl |
| 36 | iso-tridecyl | $CH_2$ | S | phenyl | Cl |
| 37 | iso-tridecyl | $CH_2$ | O | 1-naphthyl | Cl |
| 38 | iso-tridecyl | $CH_2$ | O | 4-biphenyl | Cl |
| 39 | iso-tridecyl | $CH_2$ | O | 2-chlorphenyl | Cl |
| 40 | iso-tridecyl | $CH_2$ | O | 3-bromphenyl | Cl |
| 41 | iso-tridecyl | $CH_2$ | O | 4-chlorphenyl | Cl |
| 42 | iso-tridecyl | $CH_2$ | | 4-sek.-butylphenyl | Cl |
| 43 | iso-tridecyl | $CH_2$ | O | 4-tert.-butylphenyl | Cl |
| 44 | iso-tridecyl | $CH_2$ | O | 2-methoxyphenyl | Cl |
| 45 | iso-tridecyl | $CH_2$ | O | 4-methoxyphenyl | Cl |
| 46 | iso-tridecyl | $CH_2$ | O | 4-propionylphenyl | Cl |
| 47 | iso-tridecyl | $CH_2$ | O | 4-benzylphenyl | Cl |
| 48 | iso-tridecyl | $CH_2$ | O | 2-nitrophenyl | Cl |
| 49 | iso-tridecyl | $CH_2$ | O | 4-nitrophenyl | Cl |
| 50 | iso-tridecyl | $CH_2$ | O | 4-cyanophenyl | Cl |
| 51 | iso-tridecyl | $CH_2$ | O | 2,3-dichlorphenyl | Cl |
| 52 | iso-tridecyl | $CH_2$ | O | 2,4-dichlorphenyl | Cl |
| 53 | iso-tridecyl | $CH_2$ | O | 2,5-dichlorphenyl | Cl |
| 54 | iso-tridecyl | $CH_2$ | O | 2,6-dichlorphenyl | Cl |
| 55 | iso-tridecyl | $CH_2$ | O | 3,4-dichlorphenyl | Cl |
| 56 | iso-tridecyl | $CH_2$ | O | 3,5-dichlorphenyl | Cl |

| Nr. | $R^1$ | $R^2$ | Z | $R^3$ | X |
|---|---|---|---|---|---|
| 57 | iso-tridecyl | $CH_2$ | O | 2,6-dimethylphenyl | Cl |
| 58 | iso-tridecyl | $CH_2$ | O | 3,4-dimethylphenyl | Cl |
| 59 | iso-tridecyl | $CH_2$ | O | 2,3-dimethoxyphenyl | Cl |
| 60 | iso-tridecyl | $CH_2$ | O | 2-chlor-4-nitrophenyl | Cl |
| 61 | iso-tridecyl | $CH_2$ | O | 2,4,6-trichlorphenyl | Cl |
| 62 | iso-tridecyl | $CH_2$ | O | 2,3,5-trichlorphenyl | Cl |
| 63 | iso-tridecyl | $CH_2$ | O | 2,3,6-trimethylphenyl | Cl |
| 64 | iso-tridecyl | $CH_2$ | O | 2,6-dibrom-4-nitro-phenyl | Cl |
| 65 | iso-tridecyl | $CH_2$ | O | 2,6-dibrom-4-cyano-phenyl | Cl |
| 66 | iso-tridecyl | $CH_2$ | O | 2,4-dichlor-6-methyl-phenyl | Cl |
| 67 | iso-tridecyl | $CH_2$ | O | benzyl | Cl |
| 68 | iso-tridecyl | $CH_2$ | S | benzyl | Cl |
| 69 | iso-tridecyl | $CH_2$ | O | 4-chlorbenzyl | Cl |
| 70 | iso-tridecyl | $CH_2$ | O | 4-brombenzyl | Cl |
| 71 | iso-tridecyl | $CH_2$ | O | 4-methylbenzyl | Cl |
| 72 | iso-tridecyl | $CH_2$ | O | 4-tert.-butylbenzyl | Cl |
| 73 | iso-tridecyl | $CH_2$ | O | 3-methoxybenzyl | Cl |
| 74 | iso-tridecyl | $CH_2$ | O | 4-nitrobenzyl | Cl |
| 75 | iso-tridecyl | $CH_2$ | O | 4-cyanobenzyl | Cl |
| 76 | iso-tridecyl | $CH_2$ | O | 2,4-dichlorbenzyl | Cl |
| 77 | iso-tridecyl | $CH_2$ | O | 2,6-dichlorbenzyl | Cl |
| 78 | iso-tridecyl | $CH_2$ | O | 2,3,4-trichlorbenzyl | Cl |
| 79 | iso-tridecyl | $CH_2$ | O | 2,3,6-trichlorbenzyl | Cl |
| 80 | iso-tridecyl | $CH_2$ | O | 3-phenylpropyl | Cl |
| 81 | iso-tridecyl | $CH_2-CO-O^-$ | | | |

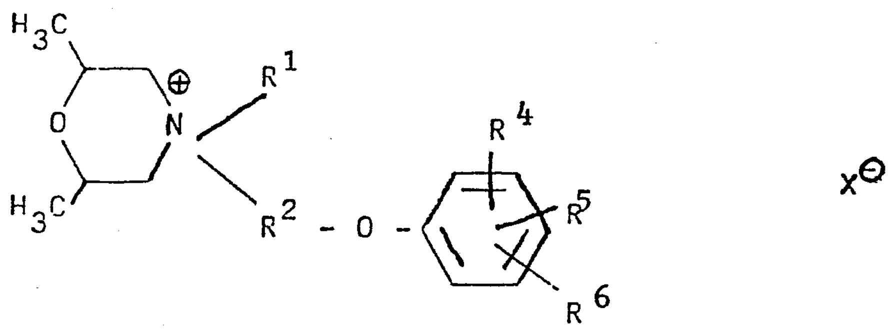

(I)

| Nr. | $R^1$ | $R^2$ | $R^4$ | $R^5$ | $R^6$ | X |
|---|---|---|---|---|---|---|
| 82 | n-octyl | $(CH_2)_2$ | 2-Cl | 6-Cl | H | Br |
| 83 | n-dodecyl | $(CH_2)_3$ | 4-Cl | H | H | Br |
| 84 | n-tridecyl | $(CH_2)_3$ | 4-Cl | H | H | Br |
| 85 | 1,5.9-tri-methyldecyl | $(CH_2)_3$ | 4-Cl | H | H | Br |
| 86 | iso-tridecyl | $(CH_2)_2$ | H | H | H | Br |
| 87 | iso-tridecyl | $(CH_2)_2$ | 4-Cl | H | H | Cl |
| 88 | iso-tridecyl | $(CH_2)_2$ | 3-F | H | H | Br |
| 89 | iso-tridecyl | $(CH_2)_2$ | 4-tert.-butyl | H | H | Cl |
| 90 | iso-tridecyl | $(CH_2)_3$ | 4-tert.-butyl | H | H | Br |
| 91 | iso-tridecyl | $(CH_2)_2$ | 4-$OCH_3$ | H | H | Br |
| 92 | iso-tridecyl | $(CH_2)_3$ | 3-$CH_3$ | H | H | Br |
| 93 | iso-tridecyl | $(CH_2)_2$ | 4-CO-$CH_3$ | H | H | Br |
| 94 | iso-tridecyl | $(CH_2)_2$ | 3-NH-CO-$CH_3$ | H | H | Br |
| 95 | iso-tridecyl | $(CH_2)_2$ | 3-CO-$OC_2H_5$ | H | H | Br |
| 96 | iso-tridecyl | $(CH_2)_2$ | 3-CO-$N(C_2H_5)_2$ | H | H | Br |
| 97 | iso-tridecyl | $(CH_2)_2$ | 4-$NO_2$ | H | H | Br |
| 98 | iso-tridecyl | $(CH_2)_2$ | 4-CN | H | H | Br |
| 99 | iso-tridecyl | $(CH_2)_2$ | 4-phenyl | H | H | Cl |
| 100 | iso-tridecyl | $(CH_2)_2$ | 4-benzyl | H | H | Cl |
| 101 | iso-tridecyl | $(CH_2)_2$ | 2-Br | 4-Br | H | Br |
| 102 | iso-tridecyl | $(CH_2)_2$ | 2-Cl | 3-Cl | H | Br |
| 103 | iso-tridecyl | $(CH_2)_3$ | 2-Cl | 4-Cl | H | Br |
| 104 | iso-tridecyl | $(CH_2)_3$ | 2-Cl | 5-Cl | H | Br |

0209763

| Nr. | $R^1$ | $R^2$ | $R^4$ | $R^5$ | $R^6$ | X |
|---|---|---|---|---|---|---|
| 105 | iso-tridecyl | $(CH_2)_2$ | 2-Cl | 6-Cl | H | Br |
| 106 | iso-tridecyl | $(CH_2)_2$ | 3-Cl | 4-Cl | H | Br |
| 107 | iso-tridecyl | $(CH_2)_3$ | $2-CH_3$ | $6-CH_3$ | H | Br |
| 108 | iso-tridecyl | $(CH_2)_2$ | $3-CH_3$ | $4-CH_3$ | H | Br |
| 109 | iso-tridecyl | $(CH_2)_2$ | 3-Cl | 5-Cl | H | Br |
| 110 | iso-tridecyl | $(CH_2)_2$ | 2-Cl | $4-NO_2$ | H | Br |
| 111 | iso-tridecyl | $(CH_2)_2$ | 2-Cl | 4-Cl | 6-Cl | Br |
| 112 | iso-tridecyl | $(CH_2)_2$ | $2-CH_3$ | $4-CH_3$ | $6-CH_3$ | Br |
| 113 | iso-tridecyl | $(CH_2)_2$ | $2-CH_3$ | 4-Cl | $6-CH_3$ | Br |
| 114 | iso-tridecyl | $(CH_2)_4$ | $2-CH_3$ | 4-Cl | $6-CH_3$ | Br |
| 115 | iso-tridecyl | $(CH_2)_2$ | 2-Cl | 4-CN | 6-Cl | Br |
| 116 | iso-tridecyl | $(CH_2)_2$ | 2-Cl | $4-NO_2$ | 6-Cl | Br |
| 117 | iso-tridecyl | $(CH_2)_3$ | 2-Br | 4-SCN | 6-Br | Br |
| 118 | $n-C_{15}H_{31}$ | $(CH_2)_2$ | 4-Cl | H | H | Br |
| 119 | $n-C_{20}H_{41}$ | $(CH_2)_2$ | 4-Cl | H | H | Br |

| Verb. Nr. | IR-Spektren von erfindungsgemäßen Verbindungen (Film) [$cm^{-1}$] |
|---|---|
| 5 | 2945, 2915, 2860, 1740, 1445, 1390, 1360, 1310, 1195, 1165, 1130, 1100, 1020, 860 |
| 8 | 2955, 2930...2910, 2870, 2680, 2610, 2505, 1735, 1455, 1370, 1325, 1170, 1125, 1080, 1040, 870, 830 |
| 14 | 2970, 2940, 2880, 2450, 1750, 1470, 1385, 1215, 1150, 1120, 1065, 1030, 880 |
| 16 | 2950, 2920, 2865, 1735, 1450, 1365, 1200, 1160, 1070, 1070, 1025, 860 |
| 24 | 2970, 2940, 2885, 2260, 1755, 1470, 1390, 1220, 1180, 890, 850 |
| 35 | 2950, 2925, 2865, 1735, 1595, 1585, 1490, 1460, 1370, 1260, 1215, 870 |
| 41 | 2960, 2930, 2875, 1750, 1605, 1595, 1465, 1440, 1385, 1270, 1225, 1095, 1030, 875, 835 |
| 49 | 2960, 2930, 2870, 1745, 1610, 1595, 1510, 1500, 1460, 1380, 1335, 1290, 1230, 1165, 1110, 850, 750, 690, 630 |
| 55 | 2950, 2920, 2865, 1740, 1590, 1565, 1470, 1425, 1375, 1320, 1280, 1210, 1115, 1080, 1015, 900, 850, 810 |
| 65 | 2950, 2920, 2860, 2220, 1735, 1590, 1575, 1560, 1530, 1460, 1370, 1285, 1230, 1190, 1125, 1080, 870 |

| Verb.-Nr. | IR-Spektren von erfindungsgemäßen Verbindungen (Film) [$cm^{-1}$] |
|---|---|
| 82 | 2945, 2920, 2850, 2615, 1555, 1440, 1365, 1320, 1235, 1190, 1170, 1100, 1040, 1030, 980, 890, 865, 825 |
| 86 | 2950, 2925, 2870, 2600, 2410, 1595, 1495, 1460, 1380, 1330, 1240, 1175, 1150, 1130, 1080, 1010, 920, 895, 875, 835 |
| 87 | 3020, 2960, 2870, 2600, 2470, 1590, 1495, 1465, 1380, 1215, 1180, 1155, 1135, 1085, 1005, 925, 875, 825 |
| 90 | 2950, 2920, 2860, 2670, 2600, 2485, 1605, 1460, 1365, 1330, 1290, 1245, 1175, 1145, 1130, 1110, 1080, 1050, 1020, 875, 825 |
| 98 | 2945, 2930, 2870, 2610, 2225, 1510, 1460, 1380, 1300, 1255, 1170, 1115, 1085, 1050, 1010, 875, 840 |
| 105 | 2960, 2930, 2870, 2610, 1565, 1450, 1375, 1325, 1245, 1175, 1140, 1065, 1045, 990, 905, 870 |
| 106 | 2950, 2920, 2870, 2690, 2680, 2620, 2480, 1590, 1570, 1470, 1385, 1330, 1280, 1225, 1175, 1120, 1085, 1060, 1015, 955, 870, 860, 835, 800 |
| 107 | 2960, 2925, 2870, 2680, 2610, 2490, 1585, 1470, 1380, 1325, 1260, 1195, 1175, 1130, 1085, 1050, 870, 830 |
| 113 | 2955, 2920, 2865, 2680, 2610, 2490, 2225, 1525, 1455, 1375, 1325, 1255, 1175, 1130, 1080, 1020, 870, 830 |

Die erfindungsgemäßen Verbindungen der allgemeinen Formel I können beispielsweise in Form folgender Zubereitungen zur Anwendung kommen:

I. Beispiel

Lösungskonzentrate: Man vermischt 80 Gewichtsteile der Verbindung 5 mit 20 Gewichtsteilen N-Methyl-2-pyrrolidon. Es wird eine Lösung erhalten, die zur Anwendung in Form kleinster Tropfen geeignet ist.

II. Beispiel

Emulgierbare Konzentrate: 25 Gewichtsteile der Verbindung 55 werden mit 2,5 Gewichtsteilen epoxydierten Pflanzenöles, 10 Gewichtsteilen eines Alkylarylsulfonat/Fettalkoholpolyglykolether-Gemisches, 5 Gewichtsteilen Dimethylformamid und 57,5 Gewichtsteilen Xylen vermischt. Aus diesem Konzentrat können durch Verdünnen mit Wasser Emuslionen jeder gewünschten Konzentration hergestellt werden.

III. Beispiel

Spritzpulver: 40 Gewichtsteile der Verbindung 65 werden mit 5 Gewichtsteilen des Natrium-Salzes einer Ligninsulfonsäure aus einer Sulfitablauge, 1 Gewichtsteil Natrium-Salz der Di-iso-butylnaphthalen-sulfonsäure und 54 Gewichtsteilen Kieselsäuregel gut vermischt und in einer entsprechenden Mühle vermahlen. Man erhält ein Spritzpulver, das mit Wasser zu Suspensionen jeder gewünschten Konzentration verdünnt werden kann.

IV. Beispiel

Stäubemittel: 5 Gewichtsteile der Verbindung 55 werden mit 95 Gewichtsteilen feinteiligen Kaolin innig vermischt und vermahlen. Die Stäubemittel können in dieser Form zur Anwendung verstäubt werden.

V. Beispiel

Granulate: 5 Gewichtsteile der Verbindung 57 werden mit 0,25 Gewichtsteile Epichlorhydrin vermischt und mit 6 Gewichtsteilen Aceton gelöst. Danach werden 3,5 Gewichtsteile Polyethylenglykol und 0,25 Gewichtsteile Cetylpolyglykolether zugesetzt. Die so erhaltene Lösung wird auf Kaolin aufgesprüht. Anschließend wird das Aceton in Vakuum verdampft. Es wird ein Mikrogranulat erhalten, das in dieser Form zur Anwendung kommen kann.

Die nachfolgenden Beispiele sollen die Erfindung weiter erläutern, ohne sie jedoch einzuschränken, und die Wirkung der Verbindungen der allgemeinen Formel I belegen:

Beispiel A

Myzelwachstums-Test

Die fungizide Wirkung der Mittel gegenüber den Testpilzen wird in herkömmlicher Weise als Hemmung des radialen Myzelwachstums auf Malzagar-Nährboden (2 % Malz) in Petrischalen von 9 cm Durchmesser bei einer Inkubationstemperatur von 25 °C bestimmt. Die Wirkstoffe werden dazu in Dimethylformamid gelöst, mit Wasser verdünnt und so dem flüssigen Agar zugemischt, daß die gewünschte Wirkstoffkonzentration im Nährmedium erreicht wird. Der DMF-Gehalt übersteigt dabei 0,5 Volumenprozent nicht. Nach dem Erkalten werden die Platten beimpft. Die Auswertung erfolgt je nach Wachstumsgeschwindigkeit der Pilze, wenn die Kontrollen ohne Wirkstoffzusatz zu 70 bis 90 % des Schalendurchmessers durchwachsen sind. Es wird die prozentuale Wachstumshemmung der Wirkstoffe berechnet (Tabelle A).

Tabelle A: Wachstumshemmung von Pilzen im Myzelwachstums-Test

| | | Wachstumshemmung in Prozent | |
|---|---|---|---|
| | | Botrytis cinerea | Phytophthora cactorum |
| Verbindung Nr. | Wirkstoff-konz. | 10 µg/ml | 50 µg/ml |
| Oxycarboxin (bekannt) | | 20 | 50 |
| 5 | | 88 | 85 |
| 8 | | 91 | 48 |
| 9 | | 92 | 51 |
| 14 | | 89 | 100 |
| 16 | | 79 | 100 |
| 24 | | 91 | |
| 35 | | 78 | 67 |
| 41 | | 86 | 100 |
| 49 | | 84 | 60 |
| 55 | | 97 | 100 |

Fortsetzung Tabelle A

| Verbindung Nr. | Wirkstoff-konz. | Wachstumshemmung in Prozent Botrytis cinerea 10 µg/ml | Phytophthora cactorum 50 µg/ml |
|---|---|---|---|
| 57 | | 94 | 100 |
| 64 | | 92 | 100 |
| 65 | | 95 | 100 |
| 81 | | 78 | |
| 82 | | 22 | |
| 86 | | 90 | |
| 87 | | 87 | |
| 90 | | 85 | |
| 98 | | 86 | 100 |
| 105 | | 88 | 81 |
| 106 | | 88 | |
| 107 | | 80 | |
| 125 | | 92 | 100 |

Beispiel B

Gerstenmehltau-Test (Erysiphe graminis/Gerste)



In Röhrchen mit Sand werden Gerstenpflanzen der Sorte "Astacus" im Einblattstadium taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach dem Antrocknen des Spritzbelages werden die Pflanzen mit Konidien des Gerstenmehltaus (Erysiphe graminis var. hordei) bestäubt. Anschließend werden die Versuchspflanzen für einen Zeitraum von 2 bis 3 Stunden bei 90 bis 100 % relativer Luftfeuchtigkeit in einer Inkubationskabine und danach bei Temperaturen zwischen 20 und 22 °C und 75 bis 80 % rel. Luftfeuchtigkeit im Gewächshaus aufgestellt. Nach 7 Tagen wird der Mehltaubefall der Gerstenpflanzen bestimmt. Die nach STEPHAN (Arch. Phytopath., Pfl.-schutz 14 (1978), 163-175) erhaltenen Boniturwerte werden in den Befallsgrad nach KRÜGER (Nachr.-Bl. Pflanzenschutz DDR 1981, 145-147) umgerechnet. Daraus ergibt sich der Wirkungsgrad nach ABBOTT entsprechend

$$WG\ (in\ \%) = \frac{Befallsgrad\ (Kontrolle) - Befallsgrad\ (Variante)}{Befallsgrad\ (Kontrolle)} \times 100$$

Die Ergebnisse gehen aus der Tabelle B hervor.

Tabelle B: Wirkung gegen Erysiphe graminis an Gerste
Wirkstoffkonzentration: 10 mg/l

| Verbindung Nr. | Wirkungsgrad in Prozent |
|---|---|
| N-Methyl-N-tridecyl-2,6-dimethyl-morpholin-methosulfat (bekannt aus DE-PS 11 67 588) | 82 |
| Triforine (bekannt) | 86 |
| 5 | 88 |
| 8 | 82 |
| 9 | 87 |
| 14 | 93 |
| 24 | 97 |
| 35 | 94 |
| 55 | 88 |
| 65 | 90 |
| 5 | 96 |
| 24 | 92 |
| 25 | 93 |
| 26 | 95 |
| 34 | 97 |

Beispiel C

Weizenmehltau-Test (Erysiphe graminis/Weizen)

In Töpfen angezogene Weizenpflanzen der Sorte "Alcedo" werden im Einblattstadium taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach dem Antrocknen des Spritzbelages werden die Pflanzen mit Konidien (Sporen) des Weizenmehltaus (Erysiphe graminis car. tritici) bestäubt. Anschließend werden die Versuchspflanzen für einen Zeitraum von 2 bis 3 Stunden bei 90 bis 100 % relativer Luftfeuchtigkeit in einer Inkubationskabine und danach bei Temperaturen zwischen 20 und 22 °C und 75 bis 80 % relativer Luftfeuchtigkeit im Gewächshaus aufgestellt.
Nach 5 Tagen wird der Mehltaubefall der Weizenpflanzen bestimmt.
Der Wirkungsgrad der Mittel wird nach der in Beispiel B beschriebenen Methode berechnet.
Die Ergebnisse gehen aus Tabelle C hervor.

Tabelle C: Wirkung gegen Erysiphe graminis an Weizen
Wirkstoffkonzentration: 100 mg/l

| Verbindung Nr. | Wirkungsgrad in Prozent |
|---|---|
| N-Methyl-N-tridecyl-2,6-dimethyl-morpholinium-methosulfat (bekannt aus DE-PS 11 67 588) | 67 |
| 5 | 88 |

0209763

Beispiel D

Getreiderost-Test (Puccinia recondita/Weizen)

In Töpfen angezogene Weizenpflanzen der Sorte "Alcedo" werden im Zweiblattstadium auf die Höhe von 12 cm zurückgeschnitten. Das Sekundärblatt der Pflanzen wird entfernt. Danach werden die Weizenpflanzen mit Wirkstoffzubereitungen besprüht, die aus 20 Gewichtsteilen Wirkstoff, 10 Gewichtsteilen Polyoxyethylensorbitanmonostearat (Tween 60), 5 Gewichtsteilen Polypropylenglykol, 25 Gewichtsteilen Cyclohexanon und 40 Gewichtsteilen Toluen hergestellt und mit Wasser auf die gewünschte Wirkstoffkonzentration verdünnt werden. Nach dem Antrocknen des Spritzbelages werden die Pflanzen mit Sporen des Weizenbraunrostes (Puccinia recondita), die als Aufschwemmung in Wasser mit Tween 60-Zusatz appliziert werden, inokuliert. Anschließend werden die Versuchspflanzen für einen Zeitraum von 24 Stunden in einer Wasserdampf-gesättigten Inkubationskabine und danach bei Temperaturen von 20 bis 22 °C und bei 70 bis 80 % relativer Luftfeuchtigkeit im Gewächshaus aufgestellt.
Nach 10 Tagen wird der Rostbefall der Weizenpflanzen ermittelt.
Der Wirkungsgrad der Mittel wird nach der in Beispiel B beschriebenen Methode berechnet.
Die Ergebnisse sind in Tabelle D aufgeführt.

Tabelle D: Wirkung gegen Puccinia recondita an Weizen
Wirkstoffkonzentration: 500 mg/l

| Verbindung Nr. | Wirkungsgrad in Prozent |
|---|---|
| Triforine (bekannt) | 85 |
| 105 | 99 |
| 107 | 98 |
| 5 | 95 |

BAD ORIGINAL

Beispiel E

Gurkenmehltau-Test (Mischkultur von Erysiphe cichorarearum und Sphaerotheca fuliginea/Gurke)

In Töpfen angezogene Gurkenpflanzen der Sorte "Eva" werden im Vierblattstadium mit einer Konidiensuspension einer Mischkultur von Erysiphe cichoracearum und Sphaerotheca fuliginea inokuliert.
Nach 4 Tagen werden die Pflanzen taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach dem Antrocknen des Spritzbelages werden die Pflanzen bei einer Temperatur von 20 °C und 70 bis 80 % relativer Luftfeuchtigkeit im Gewächshaus aufgestellt.
Nach 6 bzw. 9 Tagen wird der Mehltaubefall der Gurkenpflanzen bestimmt. Die nach BOLLE (Nachrichtbl. Dt. Pflanzenschutzdienst 16 (1964), 92-94) erhaltenen Boniturwerte werden in den Befallsgrad nach KRÜGER und daraus in den Wirkungsgrad nach ABBOTT umgerechnet.
Die Ergebnisse gehen aus Tabelle E hervor.

Tabelle E: Wirkung gegen Erysiphe cichoracearum und Sphaerotheca fuliginea (Mischkultur) auf Gurken
Wirkstoffkonzentration: 500 mg/l

| Verbindung Nr. | Wirkungsgrad in Prozent<br>Tage nach der Mittelapplikation<br>6 | <br><br>9 |
|---|---|---|
| Tridemorph (bekannt) | 100 | 88 |
| 5 | 100 | 100 |

Beispiel F

Botrytis-Test (Botrytis cinerea/Ackerbohne (Vicia faba)-Blattfieder)

Abgeschnittene Blattfieder von in Töpfen angezogenen Ackerbohnenpflanzen (Vicia faba) der Sorte "Fribo" im Vierblattstadium werden mit Wirkstoffzubereitungen bestrichen, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach Antrocknen des Spritzbelages werden die Blätter mit einer Konidiensuspension von Botrytis cinerea inokuliert, die durch Abschwemmen von 12 bis 16 Tage alten Pilzkulturen auf Malzagar-Nährmedium (2 % Malz) gewonnen wird. Die Ackerbohne-Blattfieder werden in einer Inkubationskabine in Schalen bei einer Temperatur von 22 °C und 90 bis 100 % relativer Luftfeuchtigkeit gehalten.
Nach 4 Tagen wird der Botrytis-Befall der Ackerbohne-Blattfieder bestimmt. Der auf die gesamte Blattfläche bezogene prozentuale Befall wird in den Wirkungsgrad der Mittel nach ABBOTT umgerechnet.
Die Ergebnisse gehen aus Tabelle F hervor.

Tabelle F: Wirkung gegen Botrytis cinerea auf Ackerbohne (Vicia faba)-Blattfieder
Wirkstoffkonzentration: 100 mg/l

| Verbindung Nr. | Wirkungsgrad in Prozent |
|---|---|
| Tridemorph (bekannt) | 42 |
| 5 | 45 |
| 14 | 68 |
| 55 | 48 |
| 105 | 62 |
| 106 | 47 |
| 107 | 51 |

Beispiel G

Phytophthora-Test (Phytophthora infestans/Tomate)

In Töpfen angezogene Tomatenpflanzen der Sorte "Tamina" werden im Dreiblattstadium taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach Antrocknen des Spritzbelages werden die Tomatenpflanzen mit einer wäßrigen Zoosporensuspension von Phytophthora infestans inokuliert. Anschließend werden die Versuchspflanzen in einer Inkubationskammer bei Temperaturen zwischen 18 und 20 °C und 95 bis 100 % relativer Luftfeuchtigkeit aufgestellt.
Nach 5 Tagen wird der Phytophthora-Befall der Tomatenpflanzen bestimmt. Die erhaltenen Boniturwerte werden in den Befallsgrad und den Wirkungsgrad der Mittel, wie im Beispiel B beschrieben, umgerechnet.
Die Ergebnisse sind in Tabelle G aufgeführt.

Tabelle G: Wirkung gegen Phytophthora infestans auf Tomaten

| Verbindung Nr. | Wirkstoffkonzentration in mg/l | Wirkungsgrad in Prozent |
|---|---|---|
| Zineb (bekannt) | 100 | 57 |
| | 200 | 61 |
| 105 | 100 | 66 |
| | 200 | 79 |

Beispiel H

Pflanzenwachstumsregulations-Test

Gurkenpflanzen der Sorte "Eva" werden in Töpfen in Humuserde, welche ausreichend mit Nährstoffen versorgt ist, bis zu einer Wuchshöhe von 9 cm im Gewächshaus herangezogen. Pro Versuchsvariante werden 10 Pflanzen verwendet. Die Gurkenpflanzen werden mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden.
Nach einer Wachstumszeit von 14 Tagen nach der Mittelapplikation werden an den behandelten Pflanzen und den unbehandelten Kontrollpflanzen Längenmessungen vorgenommen.
Die Ergebnisse gehen aus Tabelle H hervor.

Tabelle H: Wirkung auf das Längenwachstum von Gurkenpflanzen bei Blattbehandlung
Wirkstoffkonzentration: 1000 mg/l

| Verbindung Nr. | Pflanzenhöhe in cm | Pflanzenhöhe relativ |
|---|---|---|
| Unbehandelte Kontrolle | 23 | 100 |
| 5 | 15,6 | 68 |
| . | . | . |
| 105 | 15 | 65 |

## Patentansprüche

1. N-Alkyl-2,6-dimethylmorpholinio-carbonsäureester- und N-Alkyl-2,6-dimethylmorpholinio-alkylphenylether-Salz der allgemeinen Formel I,

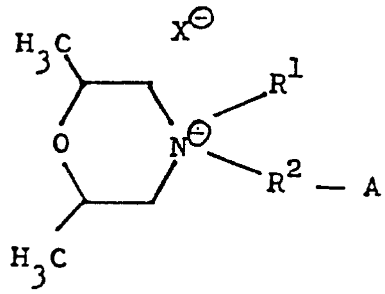

(I) ,

worin A für die Gruppierung

$-CO-Z-R^3$ oder 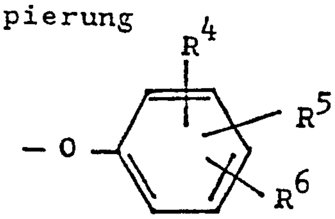 steht,

in der

$R^1$ geradkettiges oder verzweigtes Alkyl mit 6 bis 20 C-Atomen,

$R^2$ geradkettiges oder verzweigtes Alkylen mit 1 bis 6 C-Atomen,

$R^3$ geradkettiges oder verzweigtes Alkyl mit 1 bis 20 C-Atomen, ein durch Halogen, Hydroxy, Alkoxy mit 1 bis 4 C-Atomen, Dialkylamino mit 2 bis 16 C-Atomen, Nitro und/oder Cyano substituiertes geradkettiges oder verzweigtes Alkyl mit 1 bis 6 C-Atomen,
ein gegebenenfalls durch Halogen substituiertes Alkenyl mit 2 bis 6 C-Atomen,
Alkinyl mit 3 bis 6 C-Atomen,
Cycloalkyl mit 3 bis 12 Kohlenstoffatomen, wobei der Cycloalkylrest gegebenenfalls durch ein oder mehrere Alkylreste mit 1 bis 7 C-Atomen substituierte sein kann,

Cycloalkylalkyl mit 4 bis 10 C-Atomen,
Aryl, welches einfach oder mehrfach, gleich oder verschieden durch geradkettiges oder verzweigtes Alkyl mit 1 bis 6 C-Atomen, Alkoxy mit 1 bis 4 C-Atomen, Aryl-nieder-alkyl mit 7 bis 12 C-Atomen, Acyl mit 1 bis 4 C-Atomen, Halogen, Aryl, Nitro und/oder Cyano substituiert sein kann,
Aryl-nieder-alkyl mit 7 bis 12 C-Atomen, welches einfach oder mehrfach, gleich oder verschieden durch geradkettiges oder verzweigtes Alkyl mit 1 bis 6 C-Atomen, Alkoxy mit 1 bis 4 C-Atomen, Halogen, Halogenalkyl mit 1 bis 4 C-Atomen, Nitro und/oder Cyano substituiert sein kann,

$R^4$, $R^5$ und $R^6$ unabhängig voneinander gleich oder verschieden Wasserstoff, geradkettiges oder verzweigtes Alkyl mit 1 bis 6 C-Atomen, Alkoxy mit 1 bis 4 C-Atomen, Acyl mit 1 bis 4 C-Atomen Halogen, Halogenalkyl mit 1 bis 4 C-Atomen, Cycloalkyl mit 3 bis 7 C-Atomen, Aryl-nieder-alkyl mit 7 bis 12 C-Atomen,

Aryl, Nitro, Cyano, Thiocyanato, NHCOR', COOR' und/oder CONR'R'', wobei R' und R'' unabhängig voneinander für Wasserstoff, geradkettiges oder verzweigtes Alkyl mit 1 bis 6 C-Atomen, Aryl oder Aryl-nieder-alkyl mit 7 bis 12 C-Atomen steht,

Z Sauerstoff oder Schwefel und
$X^-$ das Anion einer nicht phytotoxischen Säure bedeuten, oder
$R^3$ und $X^-$ nicht vorhanden sind.

2. Verbindungen gemäß Anspruch 1, dadurch gekennzeichnet, daß $R^2$ $CH_2$ oder $CH(CH)_3$, $R^3$ $C_1$-$C_{14}$-Alkyl und Z Sauerstoff bedeuten.
3. Verbindungen gemäß Anspruch 1, dadurch gekennzeichnet, daß $R^2$ $CH_2$, $R^3$ Halogenphenyl und Z Sauerstoff bedeuten.
4. Verbindungen gemäß Anspruch 1, dadurch gekennzeichnet, daß $R^2$ $CH_2$, $R^3$ Alkylphenyl mit 7 bis 12 C-Atomen und Z Sauerstoff bedeuten.

5. Verfahren zur Herstellung von N-Alkyl-2,6-dimethylmorpholinio-carbonsäureester- und N-Alkyl-2,6-dimethylmorpholinio-alkyl-phenylether-Salzen der allgemeinen Formel I, dadurch gekennzeichnet, daß man

a) ein N-Alkyl-2,6-dimethylmorpholin der Formel II,

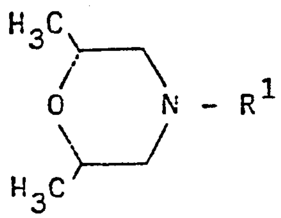

(II)

worin $R^1$ die in der allgemeinen Formel I angegebene Bedeutung besitzt,
mit einer Verbindung der Formel III,

$$X - R^2 - A \qquad (III)$$

worin $R^2$ und A die in der allgemeinen Formel I angegebene Bedeutung besitzen und X für Halogen steht,
umsetzt oder

b) einen 2,6-Dimethylmorpholino-carbonsäureester oder 2,6-Dimethylmorpholino-alkyl-phenylether der Formel IV,

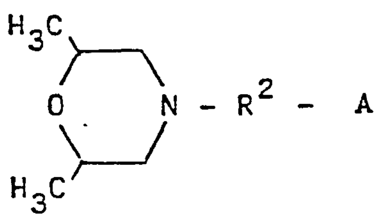

(IV)

worin $R^2$ und A die in der allgemeinen Formel I angegebene Bedeutung besitzen, mit einer Verbindung der Formel V,

$$R^1 - X \qquad (V)$$

worin $R^1$ die in der allgemeinen Formel I angegebene Bedeutung besitzt und X für Halogen steht,
umsetzt.

6. Fungizide Mittel mit zusätzlicher pflanzenwachstumsregulierender Wirkung enthaltend mindestens eine Verbindung der allgemeinen Formel I gemäß Anspruch 1.

7. Fungizide Mittel mit zusätzlicher pflanzenwachstumsregulierender Wirkung enthaltend mindestens eine Verbindung der allgemeinen Formel 1 gemäß Anspruch 1 in Mischung mit inerten Zusatzstoffen.

8. Verfahren zur Bekämpfung von Pilzen, dadurch gekennzeichnet, daß man mindestens eine Verbindung der allgemeinen Formel 1 gemäß Anspruch 1 auf Pilze oder die vor Pilzbefall zu schützenden Gegenstände einwirken läßt.

9. Verwendung von Verbindungen der allgemeinen Formel 1 gemäß Anspruch 1 zur Bekämpfung von Pilzen und/oder zur Regulierung des Pflanzenwachstums.

Europäisches Patentamt

# EUROPÄISCHER RECHERCHENBERICHT

0209763

Nummer der Anmeldung

EP 86 10 8916

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | EP-A-0 017 209 (BASF AG)<br>* Ansprüche 1-6; Seite 19, Verbindung 47; Seite 21, Verbindung 64 * | 1,5-7, 9 | C 07 D 265/30<br>A 01 N 43/84 |
| A | EP-A-0 074 005 (BASF AG)<br>* Ansprüche; Seite 3, Zeile 19 - Seite 4, Zeile 22 * | 1,5-8 | |
| A | EP-A-0 142 799 (BASF AG)<br>* Ansprüche 1-4, 6 * | 1,6-8 | |
| A | DE-A-2 915 250 (BASF AG)<br>* Ansprüche 1-3; Seite 11, Formelschema * | 1,5,6 | **RECHERCHIERTE SACHGEBIETE (Int. Cl 4)** |
| D,A | DD-A- 201 371 (F. FRANKE et al.)<br>* Ansprüche; Tabelle 1 * | 1,6,7 | A 01 N 43/84<br>C 07 C 85/04<br>C 07 D 265/00<br>C 07 D 295/00 |
| D,A | DE-B-1 167 588 (BASF AG)<br>* gesamtes Dokument * | 1,6 | |
| A | DE-B-2 036 896 (MURPHY CHEMICAL LTD.)<br>* Ansprüche 1, 7 * | 1,9 | |
| | --- -/- | | |

Der vorliegende Recherchenbericht wurde für alle Patentansprüche erstellt.

| Recherchenort | Abschlußdatum der Recherche | Prüfer |
|---|---|---|
| BERLIN | 30-09-1986 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0209763

Europäisches Patentamt

# EUROPÄISCHER RECHERCHENBERICHT

Nummer der Anmeldung

EP 86 10 8916

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB-A-1 102 011 (RICHARDSON-MERRELL S.P.A.) * Anspruch 1; Tabelle B, Seite 5, Verbindungen FC 421, FC 448, Seite 6, Verbindung FC 440 * ----- | 1 | |
| | | | RECHERCHIERTE SACHGEBIETE (Int Cl 4) |

Der vorliegende Recherchenbericht wurde für alle Patentansprüche erstellt

| Recherchenort | Abschlußdatum der Recherche | Prüfer |
|---|---|---|
| BERLIN | 30-09-1986 | HASS C V F |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument